(19) Europäisches Patentamt / European Patent Office / Office européen des brevets

(11) **EP 3 238 765 B1**

(12) **EUROPÄISCHE PATENTSCHRIFT**

(45) Veröffentlichungstag und Bekanntmachung des Hinweises auf die Patenterteilung:
**18.05.2022 Patentblatt 2022/20**

(21) Anmeldenummer: **17172308.3**

(22) Anmeldetag: **10.07.2012**

(51) Internationale Patentklassifikation (IPC):
**A61M 1/36** (2006.01)

(52) Gemeinsame Patentklassifikation (CPC):
**A61M 1/3643; A61M 1/3644;** A61M 1/3606;
A61M 2205/3306; A61M 2205/3368;
A61M 2205/3375; A61M 2205/3379;
A61M 2205/3393

(54) **VORRICHTUNGEN ZUM ERMITTELN EINES FÜLLVOLUMENS EINES EXTRAKORPORALEN BLUTKREISLAUFS**

DEVICES FOR DETERMINING A FILLING VOLUME OF AN EXTRACORPOREAL BLOOD CIRCUIT

DISPOSITIFS POUR LA DÉTERMINATION D'UN VOLUME DE REMPLISSAGE D'UN CIRCUIT SANGUIN EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2011 DE 102011108786**
**29.07.2011 US 201161513005 P**

(43) Veröffentlichungstag der Anmeldung:
**01.11.2017 Patentblatt 2017/44**

(62) Dokumentnummer(n) der früheren Anmeldung(en) nach Art. 76 EPÜ:
**12737205.0 / 2 736 559**

(73) Patentinhaber: **Fresenius Medical Care Deutschland GmbH**
**61352 Bad Homburg (DE)**

(72) Erfinder:
• **NOACK, Joachim**
**97616 Bad Neustadt (DE)**
• **ZHANG, Wei**
**97464 Niederwerrn (DE)**

(74) Vertreter: **Bobbert & Partner Patentanwälte PartmbB**
**Postfach 1252**
**85422 Erding (DE)**

(56) Entgegenhaltungen:
**EP-A1- 0 416 808      WO-A1-2010/133319**
**US-A1- 2005 173 343**

• **NIKOLAI M. KRIVITSKI ET AL: "In vivo measurement of hemodialyzer fiber bundle volume: Theory and validation", KIDNEY INTERNATIONAL, Bd. 54, Nr. 5, 1. November 1998 (1998-11-01), Seiten 1751-1758, XP055402661, LONDON, GB ISSN: 0085-2538, DOI: 10.1046/j.1523-1755.1998.00146.x**

**Beschreibung**

Die vorliegende Erfindung betrifft eine Steuer- oder Regelungseinrichtung gemäß Anspruch 1 und eine Behandlungseinrichtung gemäß Anspruch 18.

Extrakorporale Blutkreisläufe erfahren in der Praxis vor ihrer Verwendung in einem medizinischen Behandlungsverfahren eine Vorbereitung. Die zur Behandlung mit dem extrakorporalen Blutkreislauf eingesetzte Behandlungsvorrichtung wird in der Praxis vor Behandlungsbeginn an die anstehende Behandlungsmodalität und/oder an die verwendeten (Einweg-)Artikel wie Blutkreislauf oder Blutfilter angepasst. Zu diesem Zweck wird der extrakorporale Blutkreislauf üblicherweise vor seinem Gebrauch mit einem Fluid (beispielsweise einer Substituatlösung) befüllt und hiermit gespült um möglicherweise vorhandene Produktionsrückstände und/oder Luftblasen aus dem extrakorporalen Blutkreislauf zu entfernen; an der Behandlungsvorrichtung wird gelegentlich eingestellt, mit welcher Behandlungsmodalität behandelt werden soll oder darf, oder von welchem Typ oder von welcher Größe der eingesetzte Blutfilter oder Blutkreislauf ist. Sowohl zum Bestimmen eines Mindestspülvolumens für den Blutkreislauf als auch für das Einstellen der Behandlungsvorrichtung auf die verwendeten (Einweg-)Artikel ist es erforderlich, wenigstens einen Parameter hierüber zu kennen.

Krivitski et al. ("In vivo measurement of hemodialyzer fiber bundle volume: theory and validation"; Kidney International; Bd. 54, Nr. 5, 1998, Seiten 1751-1758) offenbart ein Verfahren zum Ermitteln des Volumens eines Dialysators.

EP0416808 A1 offenbart eine Vorrichtung zur Messung des Flusses in einer Leitung.

WO2010/133319 offenbart eine Vorrichtung und ein Verfahren zur Erkennung eines Schlauchleitungssystems für eine extrakorporale Blutbehandlungsvorrichtung.

Eine Aufgabe der vorliegenden Erfindung ist es, eine Steuer- oder Regelungseinrichtung und eine Behandlungsvorrichtung, mit welcher das offenbarte Verfahren zum, vorzugsweise automatischen, Ermitteln wenigstens eines Parameters eines extrakorporalen Blutkreislaufs durchführbar ist, anzugeben.

Die erfindungsgemäße Aufgabe wird durch eine Steuer- oder Regelungseinrichtung mit den Merkmalen des Anspruchs 1 gelöst. Sie wird zudem durch eine Behandlungsvorrichtung mit den Merkmalen des Anspruchs 18

Die erfindungsgemäße Steuer- oder Regelungseinrichtung ist konfiguriert zur Durchführung eines Verfahrens zum Ermitteln wenigstens eines Parameters eines extrakorporalen Blutkreislaufs, wobei der extrakorporale Blutkreislauf eine Behandlungseinrichtung aufweist, mit den Schritten:

- Befüllen eines extrakorporalen Blutkreislaufs durch Einbringen eines Fluids, Fördern des Fluides durch den extrakorporalen Blutkreislauf oder Abschnitte hiervon, mittels einer Blutpumpe bei konstantem und/oder vorbestimmtem Fluss ($Q\_B$) der Blutpumpe, und Bestimmen oder Errechnen einer Laufzeit des mittels der Blutpumpe in den extrakorporalen Blutkreislauf, oder Abschnitte hiervon, geförderten Fluids; und
- Erfassen, Ermitteln oder Berechnen der Größe eines Volumens des eingebrachten Fluids, welches zum Befüllen des extrakorporalen Blutkreislaufs oder eines definierten oder vorbestimmten Abschnitts hiervon erforderlich ist;
- Weiterverarbeiten der Größe des erfassten Volumens des Fluids zum Erhalt des wenigstens einen Parameters des extrakorporalen Blutkreislaufs; wobei der Parameter eine Angabe ist, welche den extrakorporalen Blutkreislauf unterscheidbar macht, seine Typenzugehörigkeit, seine Klassenzugehörigkeit, seine Eignung für bestimmte Behandlungsmodalitäten oder -verfahren oder seine Eignung zur Verwendung mit bestimmten Behandlungsvorrichtungen angibt oder Aussagen über einzelne Komponenten hiervon macht, dadurch gekennzeichnet, dass das Verfahren den Schritt umfasst: Vergleichen des ermittelten Parameters mit Parametern einer Vielzahl einseztbarer Behandlungseinrichtungen zur Identifikation der verwendeten Behandlungseinrichtung.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Ein extrakorporaler Blutkreislauf weist in bestimmten Ausführungsformen der vorliegenden Erfindung eine medizinische Funktionseinrichtung auf. Diese Funktionseinrichtung kann beispielsweise eine (insbesondere als Disposable ausgestaltete) Blutkassette, und/oder eine Behandlungseinrichtung, wie beispielsweise einen Dialysator oder einen Blutfilter, und/oder einen Blutschlauchsatz aufweisen oder hieraus bestehen.

Die Größe des Volumens (auch kurz: das Volumen) ist in manchen erfindungsgemäßen Ausführungsformen ein Zahlenwert mit einer Dimension (wie ml; Milliliter) oder ohne Dimension. In anderen erfindungsgemäßen Ausführungsformen wird hierunter eine Klassifizierung des Volumens oder seine Zuordnung zu bestimmten Volumenklassen verstanden.

Das "Befüllen" bezeichnet in bestimmten Ausführungsformen der vorliegenden Erfindung das Einbringen oder Einfüllen von - insbesondere medizinischem - Fluid, z. B. Substituat-und/oder Dialysierflüssigkeit, in den extrakorporalen Blutkreislauf. Das Befüllen erfolgt in einigen erfindungsgemäßen Ausführungsformen einmalig und/oder initial, d. h. vor Beginn der Behandlung des Patienten, beispielsweise eines Blutbehandlungsverfahrens, z. B. einer Dialyse (Hämodialyse, Hämofiltration, Hämodiafiltration oder dergleichen) und/oder vor Beginn des Spülvorgangs.

In bestimmten Ausführungsformen der vorliegenden Erfindung ist das Befüllen im Sinne der vorliegenden Erfindung vor Beginn des Spülvorgangs begonnen, erfolgt oder abgeschlossen.

Der extrakorporale Blutkreislauf wird in manchen erfindungsgemäßen Ausführungsformen beim Befüllen vollständig oder im Wesentlichen vollständig mit Fluid befüllt.

Dabei wird in einigen erfindungsgemäßen Ausführungsformen so viel Fluid in den extrakorporalen Blutkreislauf eingebracht, dass das eingebrachte Volumen einem Innenvolumen oder einem maximalen (Aufnahme-)Volumen des extrakorporalen Blutkreislaufs (mit oder ohne hiermit in Fluidverbindung stehenden Komponenten wie einem Blutfilter oder einer Blutkassette) entspricht.

Um das Volumen des extrakorporalen Blutkreislaufs oder eines Abschnitts hiervon zu erfassen, muss der extrakorporale Blutkreislauf in manchen erfindungsgemäßen Ausführungsformen nicht vollständig mit Fluid befüllt werden.

So wird in bestimmten Ausführungsformen der vorliegenden Erfindung der extrakorporale Blutkreislauf zum Beispiel nur teilweise, beispielsweise bis zu einem bestimmten Punkt, z. B. einer im oder am extrakorporalen Blutkreislauf angeordneten Erkennungseinrichtung, eingesetzt zum Erkennen eines Fluids oder zum Erkennen eines Wechsels zwischen unterschiedlichen Fluiden, mit Fluid befüllt.

In manchen Ausführungsformen kann beispielsweise vorgesehen sein, das (Füll-)Volumen oder Gesamtvolumen oder Gesamtinnenvolumen oder Fassungsvermögen des extrakorporalen Blutkreislaufs hochzurechnen oder abzuschätzen. Diese Hochrechnung oder Abschätzung stützt sich auf die Größe des erfassten Volumens (beispielsweise ist dies der befüllte Abschnitt des extrakorporalen Blutkreislaufs zwischen dem Einbringungsort des Fluids und dem Erkennungsort) und auf die Kenntnis des verbleibenden Teils des gesamten extrakorporalen Blutkreislaufs.

Das in den extrakorporalen Blutkreislauf eingebrachte Fluid ist in manchen erfindungsgemäßen Ausführungsformen eine Priming- und/oder Spülflüssigkeit, beispielsweise eine Substituatflüssigkeit.

In allen Ausführungsformen umfasst das Verfahren das Weiterverarbeiten der erfassten Größe des Volumens des Fluids. Das Weiterverarbeiten dient in den erfindungsgemäßen Ausführungsformen zum Erhalten des wenigstens einen Parameters des extrakorporalen Blutkreislaufs.

Das Weiterverarbeiten der erfassten Größe ist oder umfasst in einigen erfindungsgemäßen Ausführungsformen ein Treffen einer Aussage über den Parameter.

Das Weiterverarbeiten der erfassten Größe des Volumens ist oder umfasst in einigen erfindungsgemäßen Ausführungsformen ein Umrechnen des Wertes des Volumens über Algorithmen, Funktionen oder Zusammenhänge zum Erhalt des Parameters. Sie können in einer Speichereinrichtung, z. B. einer Speicheroder Steuereinrichtung einer Behandlungsvorrichtung, hinterlegt sein oder werden.

Das Weiterverarbeiten der erfassten Größe des Volumens ist oder umfasst in manchen erfindungsgemäßen Ausführungsformen ein Mitteilen des erhaltenen Parameters an eine Aufsichtsperson, in anderen Ausführungsformen hingegen nicht. Das Weiterverarbeiten der erfassten Größe des Volumens ist oder umfasst in bestimmten erfindungsgemäßen Ausführungsformen ein Vergleichen des erfassten Volumens mit zur Ermittlung des Parameters geeigneten Größen oder Angaben (beispielsweise anhand einer Vergleichstabelle).

Das Weiterverarbeiten der erfassten Größe des Volumens ist oder umfasst in gewissen erfindungsgemäßen Ausführungsformen ein Zuordnen eines Parameters zum extrakorporalen Blutkreislauf und/oder zu einzelnen Komponenten hiervon basierend auf der erfassten Größe des Volumens.

Das Weiterverarbeiten der erfassten Größe des Volumen umfasst in manchen erfindungsgemäßen Ausführungsformen ein Mitteilen des Parameters an eine Aufsichtsperson (beispielsweise mittels Displayanzeige, Fehlermeldung, Alarm oder dergleichen), in anderen nicht.

Ein Parameter ist eine Angabe, welche charakteristisch für den verwendeten extrakorporalen Blutkreislauf ist, und zwar alternativ ausschließlich (die Angabe oder der Parameter ist eindeutig nur dem verwendeten extrakorporalen Blutkreislauf oder dem verwendeten Typ von extrakorporalen Blutkreisläufen zugeordnet) oder nicht ausschließlich (die Angabe oder der Parameter ist neben dem verwendeten extrakorporalen Blutkreislauf oder dem verwendeten Typ von extrakorporalen Blutkreisläufen auch weiteren Blutkreisläufen oder Typen von extrakorporalen Blutkreisläufen zugeordnet, lässt aber dennoch eine gewisse Zuordnung, etwa zu einer abgeschlossenen Gruppe von extrakorporalen Blutkreisläufen, zu).

Das Volumen des Fluids, welches zum Befüllen des extrakorporalen Blutkreislaufs in diesen eingebracht wird, ist in bestimmten Ausführungsformen der vorliegenden Erfindung ein kumuliertes Volumen.

Ein kumuliertes Volumen bezeichnet in bestimmten Ausführungsformen der vorliegenden Erfindung das gesamte zum erstmaligen oder einmaligen, insbesondere zum vollständigen oder im Wesentlichen vollständigen, Befüllen des extrakorporalen Blutkreislaufs eingebrachte Volumen an Fluid. Das kumulierte Volumen umfasst also auch Teilvolumina, welche zum Erzielen des erstmaligen Befüllens des extrakorporalen Blutkreislaufs z. B. aufgrund einer während und aufgrund des Befüllens erfolgenden Entlüftung des extrakorporalen Blutkreislaufs zum Erzielen des angestrebten Befüllens ersetzt oder nachgefüllt werden müssen.

Das Erreichen des befüllten Zustands des extrakorporalen Blutkreislaufs wird mittels einer Erkennungseinrichtung zum Erkennen eines Fluids oder eines Fluidzustands (wie einer Färbung, eines Mischungsverhältnisses, eines Wechsels unterschiedlicher Fluide, etwa eines Verdrängens von Luft durch das Fluid, einer Grenze zwischen einem flüssigen Fluid und Luft, usw.) festgestellt.

Die Erkennungseinrichtung ist in gewissen Ausführungsformen der vorliegenden Erfindung im oder am extrakorporalen Blutkreislauf angeordnet. In einigen erfindungsgemäßen Ausführungsformen steht sie mit diesem in Wirk- oder Signalverbindung.

In bestimmten Ausführungsformen der vorliegenden Erfindung ist die Erkennungseinrichtung ausgewählt aus Pegeldetektoren (beispielsweise Ultraschall-Pegeldetektoren, kapazitiven Pegeldetektoren, optischen Pegeldetektoren oder dergleichen), Luftdetektoren (beispielsweise Luftblasendetektoren), venösen Blasenfängern, oder dergleichen, oder Kombinationen hiervon, welche nach demselben physikalischen Prinzip oder nach unterschiedlichen Prinzipien arbeiten.

In allen Ausführungsformen der vorliegenden Erfindung wird eine Laufzeit eines mittels einer Blutpumpe in extrakorporalen Blutkreislauf geförderten Fluids (welches in diesen Ausführungsformen beispielsweise Dialysierflüssigkeit, Substituatflüssigkeit oder Blut ist) bestimmt oder errechnet. In diesen Ausführungsformen umfasst das Verfahren das Fördern des Fluids durch den extrakorporalen Blutkreislauf oder Abschnitte hiervon mittels einer Pumpe, beispielsweise der Blutpumpe. Diese fördert entsprechend eines gewünschten konstanten Flusses oder eines nichtkonstanten aber auf andere Weise vorbestimmten Flussverlaufs, welcher eine Berechnung des innerhalb einer betrachteten Zeitspanne geförderten Fluidvolumens erlaubt. Ein solcher vorbestimmter Fluss kann beispielsweise ein sich kontinuierlich verstärkender Fluss oder ein sich zu vorbestimmten Zeitpunkten sprungweise erhöhender Fluss bei bekannter Sprunghöhe sein.

In diesen Ausführungsformen wird der Zeitpunkt des Auftretens des Fluids an einem ersten Sensor des extrakorporalen Blutkreislaufs ermittelt. Dieser Zeitpunkt wird als erster Zeitpunkt bezeichnet.

In diesen Ausführungsformen wird der Zeitpunkt des Auftretens des Fluids an einem zweiten Sensor des extrakorporalen Blutkreislaufs ermittelt. Dieser Zeitpunkt wird als zweiter Zeitpunkt bezeichnet.

In diesen Ausführungsformen wird die Zeitdifferenz zwischen dem ersten Zeitpunkt und dem zweiten Zeitpunkt ermittelt.

In diesen Ausführungsformen ist das Erfassen, Ermitteln oder Berechnen der Größe des Volumens des eingebrachten Fluids eine Multiplikation der Zeitdifferenz mit der konstanten Flussrate der Blutpumpe oder umfasst eine solche Multiplikation.

In diesen Ausführungsformen kann das Volumen des extrakorporalen Blutkreislaufs somit anhand der Laufzeit des Fluids, beispielsweise des Bluts, vom ersten Sensor zum zweiten Sensor ermittelt werden, wenn die Förderleistung oder -menge der verwendeten Pumpe, hier nur beispielsweise einer Blutpumpe, bekannt ist. Die Förderleistung oder -menge der Pumpe kann sich aus einer als konstant oder anderweitig bekannten Förderrate der Pumpe zwischen dem ersten und dem zweiten Zeitpunkt ergeben.

Auf diese Weise lässt sich anhand der Zeitdifferenz oder der Laufzeit und der Pumpenrate, - leistung oder der -menge pro Zeit das Gesamtvolumen des geförderten Fluids und damit das von den Schlauchabschnitten und der Behandlungseinrichtung (z. B. einem Dialysator) gemeinsam aufgenommene Füllvolumen bestimmen.

Ist das Füllvolumen des extrakorporalen Blutkreislaufs, insbesondere der Schlauchabschnitte hiervon, bekannt, so lässt sich der verwendete Typ der Behandlungseinrichtung (z. B. des Dialysators) bestimmen, da jeder Typ einer Behandlungseinrichtung ein für ihn typisches Volumen aufnimmt.

Jeder der hierin genannten optischen Sensoren kann ein venöser Blutdetektor (zum Detektieren von Blut im venösen Schenkel des extrakorporalen Blutkreislaufs) auf optischer Basis, ein optischer Sensor zur Messung wenigstens eines Blutparameters, oder jeder andere Typ eines Sensors sein. Unter einem solchen Blutparameter sind u. a. das relative Blutvolumen, die Hämoglobinkonzentration und der Hämatokrit zu verstehen. Andere optische Detektoren, welche verwendet werden können, sind nicht zum Messen von Blutparametern ausgestaltet.

Ein wie vorstehend beschriebener optischer Sensor zur Messung wenigstens eines Blutparameters ist in gewissen Ausführungsformen der vorliegenden Erfindung eine Vorrichtung für Messungen im Blut wie sie in der Offenlegungsschrift EP 1 748 292 A1 und/oder in der Offenlegungsschrift WO 2004/057313 A1 offenbart ist.

Ein optischer Sensor zur Messung wenigstens eines Blutparameters kann insbesondere eine Vorrichtung sein, welche eine Messeinheit aufweist, die einen Lichtemitter und einen Lichtdetektor aufweist. Hiermit wird Licht mit einer vorgegebenen Wellenlänge in einen Abschnitt des extrakorporalen Blutkreislaufs eingebracht, beispielsweise in den arteriellen Abschnitt. Der Abschnitt ist für Licht, insbesondere Infrarotlicht, durchlässig. Mit dem Lichtdetektor wird aus dem Abschnitt austretendes Licht empfangen. Mittels des optischen Sensors zur Messung wenigstens eines Blutparameters kann das Vorliegen von Fluid, beispielsweise Blut, im Abschnitt erkannt werden. Dies kann beispielsweise anhand des Verhältnisses zwischen der Intensität des ein- und der Intensität des austretenden Lichts erfolgen.

In einigen erfindungsgemäßen Ausführungsformen ist der optische Sensor zur Messung wenigstens eines Blutparameters ausgestaltet, um weitere Blutparameter wie relatives Blutvolumen, Hämoglobinkonzentration, Hämatokrit zu messen. Die Steuereinrichtung ist entsprechend konfiguriert oder eingerichtet.

Wird die Anwesenheit von Fluid mittels Licht festgestellt, so ist in bestimmten erfindungsgemäßen Ausführungsformen die Verwendung von Licht mit einer Wellenlänge von 790 bis 820 nm, bevorzugt von 800 bis 810 nm und besonders bevorzugt von 805 nm vorgesehen.

Soll die Anwesenheit von Fluid mittels Licht festgestellt werden, so sind in manchen erfindungsgemäßen Ausführungsformen der Lichtemitter und der Lichtdetektor an einem Abschnitt des extrakorporalen Blutkreislaufs für eine Messung in diesem Abschnitt oder durch diesen hindurch angeordnet, welcher zwischen der arteriellen Patientennadel und der Blutpumpe liegt.

Dieser Abschnitt ist in gewissen erfindungsgemäßen Ausführungsformen steifer (hierin auch als stabiler geführt oder aufgenommen bezeichnet) als wenigstens ein anderer Abschnitt des extrakorporalen Blutkreislaufs, als ein stromabwärts der Blutpumpe angeordneter Abschnitt, oder als alle anderen Abschnitte des extrakorporalen Blutkreislaufs. Der steifere Abschnitt ist in manchen erfindungsgemäßen Ausführungsformen ein Abschnitt, welcher zumindest teilweise an seinem Äußeren oder Umfang von einer Begrenzung oder Manschette (im Folgenden kurz: Manschette) umgeben ist. Die Manschette übt in gewissen erfindungsgemäßen Ausführungsformen Druck auf den durch sie hindurch geführten Abschnitt aus, beispielsweise derart, dass der Abschnitt im Inneren der Manschette ein anderes Querschnittprofil aufweist als vor und/oder hinter der Manschette. Aus diesem Grund kann er als steif(er) bezeichnet werden. Dies kann die Messgenauigkeit mittels Lichtdetektor vorteilhaft erhöhen. Beispielsweise kann der an sich runde oder kreisförmige Querschnitt des Blutschlauchs in der Manschette in einen eher eckigen oder viereckigen Querschnitt oder einen Querschnitt mit wenigstens einer oder zwei geraden Seitenflächen geformt werden. Ein Beispiel einer solchen Manschette oder der ihr zugrunde liegenden Idee ist in den oben genannten Offenlegungsschriften EP 1 748 292 A1 und WO 2004/057313 A1 offenbart, siehe beispielsweise jeweils die Fig. 1 bis 5 dort.

Insbesondere kann die Pressung des Blutschlauchs durch die Manschette einen vorbestimmten Druck und/oder eine vorbestimmte Form- oder Querschnittsveränderung bewirken. Der Blutschlauchabschnitt kann somit auf definierte Weise verpresst oder gepresst sein.

Insbesondere kann die Manschette in gewissen erfindungsgemäßen Ausführungsformen ausgestaltet sein als ein längliches Behältnis.

Insbesondere kann die Manschette in gewissen erfindungsgemäßen Ausführungsformen ausgestaltet sein mit Aufnahmen oder Durchlässen für Lichtemitter und/oder Lichtdetektor.

Sowohl der erste Sensor als auch der zweite Sensor können unabhängig voneinander als Sensoren ausgestaltet sein, welche mittels Messung der optischen Dichte, Ultraschallmessung, Akustikmessung oder Temperaturmessung die Anwesenheit von Fluid feststellen.

In einigen Ausführungsformen der vorliegenden Erfindung wird die ermittelte Zeitdifferenz zu Zwecken der Verfahrenssteuerung der Blutbehandlung oder des Abrüstens der Blutbehandlungsvorrichtung nach abgeschlossener Behandlung genutzt. Beispielsweise kann die Kenntnis der Zeitdifferenz für einen automatischen Stopp der Reinfusion des Blutes nach Behandlungsende verwendet werden.

In gewissen Ausführungsformen der vorliegenden Erfindung ist die Erkennungseinrichtung dazu vorgesehen, eine Fluid-Luft-Grenze zu erkennen. Sie kann somit angeben, wann die im (noch unbenutzten) extrakorporalen Blutkreislauf befindliche Luft stromaufwärts der Erkennungseinrichtung durch das eingefüllte Fluid verdrängt und beispielsweise über eine Entlüftungseinrichtung aus dem extrakorporalen Blutkreislauf entfernt wurde.

In bestimmten Ausführungsformen der vorliegenden Erfindung wird das Volumen des Fluids über die (Anzahl der) Rotordrehungen einer Fördereinrichtung, mittels welcher der extrakorporale Blutkreislauf befüllt wurde, erfasst.

Bei der Fördereinrichtung kann es sich um eine Schlauchrollenpumpe handeln, zum Beispiel eine Schlauchrollenpumpe, welche in Behandlungsverfahren zum Fördern von Blut (Blutpumpe) und/oder Substituat (Substituatpumpe) und/oder Dialysat eingesetzt wird.

In weiteren Ausführungsformen der vorliegenden Erfindung wird das Volumen des Fluids über die Gewichtsabnahme einer Quelle für das Fluid, z. B. eines Flüssigkeitsbeutels, erfasst. Die Gewichtsabnahme kann beispielsweise durch Wiegen des Flüssigkeitsbeutels erfasst werden.

In bestimmten Ausführungsformen der vorliegenden Erfindung umfasst das offenbarte Verfahren das Vergleichen des ermittelten Parameters mit (erfassten und hinterlegten) Parametern einer Vielzahl einsetzbarer medizinischer Funktionseinrichtungen zur Identifikation oder Zuordnung des extrakorporalen Blutkreislaufs einer hierin vorliegenden oder verwendeten medizinischen Funktionseinrichtung oder Komponente. Ein solches Vergleichen entspricht in manchen erfindungsgemäßen Ausführungsformen dem oben diskutierten Weiterverarbeiten oder umfasst dieses.

Das Vergleichen kann z. B. mittels einer Vergleichstabelle erfolgen.

In allen Ausführungsformen der Erfindung umfasst das Verfahren das Vergleichen des ermittelten Parameters mit (erfassten und hinterlegten) Parametern einer Vielzahl einsetzbarer Behandlungseinrichtungen zur Identifikation einer verwendeten Behandlungseinrichtung.

Die Vielzahl der einsetzbaren medizinischen Funktionseinrichtungen oder der einsetzbaren Behandlungseinrichtungen oder Informationen jeweils zu ihnen können in geeigneten Tabellen hinterlegt sein.

Ein vor einer Verwendung des extrakorporalen Blutkreislaufs zu einer Behandlung üblicherweise erfolgendes Vorbereiten, beispielsweise durch Primen und/oder Spülen dient wie oben erwähnt dazu, etwaige Produktionsrückstände (bedingt durch die Produktion der Behandlungseinrichtung und/oder des Blutschlauchsatzes) und/oder Luft aus dem Blutkreislauf oder den hiermit verbundenen Komponenten zu entfernen. Die zum Spülen oder Primen erforderliche Fluidmenge oder das erforderliche Fluidvolumen ist üblicherweise größer als die zum einfachen Befüllen des extrakorporalen Blutkreislaufs erforderliche Fluidmenge bzw. das Fassungs- oder Fluidvolumen. Im Stand der Technik wird zum Primen/Spülen ein allgemein gültiges Mindestvolumen von z. B. 500 ml festgelegt. Diese Festlegung ist offensichtlich nicht auf den jeweils konkret verwendeten extrakorporalen Blutschlauchsatz und seine Komponenten, etwa den verwendeten Blutfilter, abgestimmt, angepasst oder gar optimiert.

Das offenbarte, nicht-erfindungsgemäße Verfahren umfasst in bestimmten Ausführungsformen das Festlegen eines minimalen, optimalen und/oder maximalen Priming- oder Spülvolumens zum Primen oder Spülen des extrakorporalen Blutkreislaufs anhand des ermittelten Parameters. Damit wird das Priming- oder Spülvolumen anhand des ermittelten Parameters vorteilhaft auf den tatsächlich verwendeten extrakorporalen Blutkreislauf und seine Komponenten angepasst. Hierbei kann der Parameter beispielsweise das Füllvolumen des Blutkreislaufs oder dessen Typ sein.

In gewissen Ausführungsformen kann ein Mindestspülvolumen V_min zur Vorbereitung des später erfolgenden Behandlungsverfahrens, welches nicht Gegenstand oder Teil der vorliegenden Erfindung ist, beispielsweise der folgenden mathematischen Funktion folgen:

$$V\_min = a*V\_Set + b*V\_Dial$$

Hierbei ist V_min das Mindestspülvolumen bei der Vorbereitung des Behandlungsverfahrens; V_Set ist das Volumen der medizinischen Funktionseinrichtung, insbesondere eines Blutschlauchsatzes oder Blutschlauchsets; V_Dial ist das Volumen der Behandlungseinrichtung, insbesondere eines Dialysators oder Blutfilters; und a und b sind jeweils Zahlen oder Konstanten.

In bestimmten Ausführungsformen ist a eine beliebige Zahl, welche Werte insbesondere von 1 bis 3 annehmen kann.

In gewissen Ausführungsformen ist b eine beliebige Zahl, welche Werte insbesondere von 2 bis 5 annehmen kann.

In manchen Ausführungsformen können in die Bestimmung des Priming- oder Spülvolumens weitere Faktoren, wie beispielsweise (Behandlungs-)Vorschriften oder dergleichen, eingehen.

In bestimmten Ausführungsformen umfasst das Verfahren das Festlegen oder Beeinflussen einer Steuerung des (späteren) Behandlungsverfahrens unter Einbezug oder in Abhängigkeit des ermittelten Parameters.

In einigen Ausführungsformen umfasst das offenbarte, nicht-erfindungsgemäße Verfahren das Festlegen von maximalen und/oder minimalen Pumpenraten, ein (automatisches) Festlegen von Reinfusionsvolumina oder dergleichen unter Einbezug oder in Abhängigkeit des ermittelten Parameters.

In bestimmten erfindungsgemäßen Ausführungsformen sind Reinfusionsvolumina solche Volumina einer Flüssigkeit (z. B. Substituat, Dialysierflüssigkeit oder Spülflüssigkeit), welche erforderlich sind, um das nach Beendigung der Blutbehandlung noch im extrakorporalen Blutkreislauf enthaltene Blut aus diesem herauszuspülen mit dem Ziel, das Blut dem Patienten rückzuführen.

In gewissen Ausführungsformen umfasst das offenbarte, nicht-erfindungsgemäße

Verfahren das Sperren von Behandlungsmodalitäten und/oder das Einschränken von Behandlungsparametern des (späteren) Behandlungsverfahrens unter Einbezug oder in Abhängigkeit des ermittelten Parameters.

Einige oder alle erfindungsgemäßen Ausführungsformen können einen oder mehrere der oben oder im Folgenden genannten Vorteile aufweisen.

Die vorliegende Offenbarung stellt vorteilhaft ein einfaches und wenig aufwendiges Verfahren zur (weiteren) Automatisierung eines Behandlungsverfahrens bereit.

Die vorliegende Erfindung ist vorteilhaft für alle Behandlungsvorrichtungen mit extrakorporalen Blutkreisläufen einsetzbar.

Mittels der erfindungsgemäßen Steuer- oder Regelungseinrichtung ist es in manchen Ausführungsformen vorteilhaft möglich, basierend auf der beim initialen Befüllen eines extrakorporalen Blutkreislaufs gewonnenen Information des (Füll-)Volumens oder eines anderen Parameters wie des Typs des verwendeten Blutkreislaufs oder dergleichen eine individualisierte Vorgabe des während des nachfolgenden Spülens umzuwälzenden Flüssigkeitsvolumens zu machen. Dies kann zu einer optimalen Spülwirkung, einer Mindestspülwirkung und/oder zu einem sparsamen Verwenden an Spülfluid beitragen.

Mittels der erfindungsgemäßen Steuer- oder Regelungseinrichtung ist es in einigen Ausführungsformen vorteilhaft möglich, basierend auf der beim initialen Befüllen eines extrakorporalen Blutkreislaufs gewonnenen Information des (Füll-)Volumens gewisse Behandlungsmodalitäten zuzulassen oder zu sperren, entsprechend der erkannten Eignung, Größe, oder des Typs von extrakorporalem Blutkreislauf und/oder verwendeter Behandlungsvorrichtung. Dies kann vorteilhaft dazu beitragen, die Vorbereitung einer Behandlung und/oder den Behandlungsablauf selbst weiter zu automatisieren. Hiermit kann ferner die Sicherheit des Systems sowie die Patientensicherheit erhöht werden.

Die vorliegende Erfindung ist vorteilhaft besonders einfach implementierbar, da sie oft nur ein softwareseitiges Aufrüsten von sich bereits in Betrieb befindenden Behandlungsvorrichtungen umfasst.

Ein derartiges Aufrüsten oder Nachrüsten kann z. B. durch Implementieren des Quell- oder Maschinencodes der Betriebssoftware in der Steuereinrichtung einer Behandlungsvorrichtung erfolgen.

Im Folgenden wird die vorliegende Erfindung unter Bezugnahme auf die beigefügte Zeichnung, in welcher gleiche Bezugszeichen identische oder ähnliche Elemente bezeichnen, rein exemplarisch beschrieben. Es gilt:

Fig. 1 zeigt eine schematische Abbildung eines extrakorporalen Blutkreislaufs, welcher mittels des offenbarten, nicht-erfindungsgemäßen Verfahrens zu seiner Verwendung vorbereitet werden kann, sowie eines Dialysatkreislaufs;

Fig. 2 veranschaulicht das Füllvolumen verschiedener Dialysatoren und deren Identifizierbarkeit mittels des offenbarten, nicht-erfindungsgemäßen Verfahrens; und

Fig. 3 zeigt eine schematische Abbildung eines weiteren extrakorporalen Blutkreislaufs, welcher mittels des offenbarten, nicht-erfindungsgemäßen Verfahrens zu seiner Verwendung oder abschließenden Spülung vorbereitet werden kann.

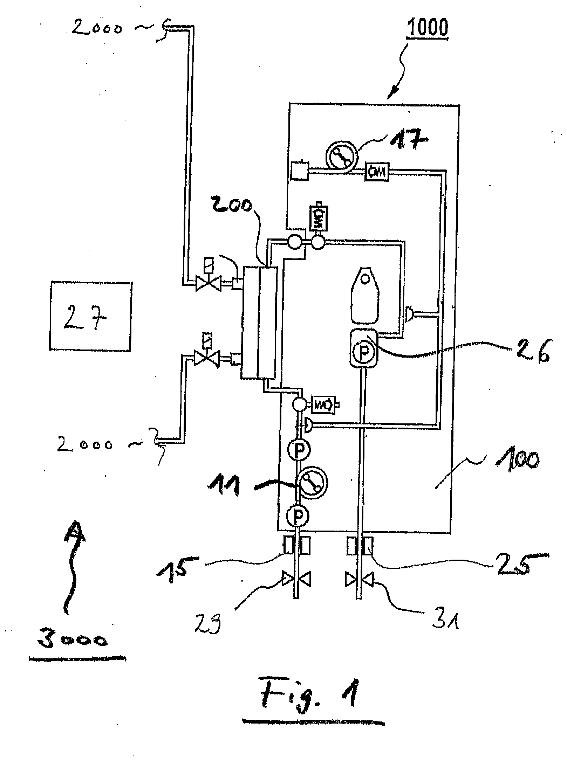

**Fig. 1** zeigt schematisch einen extrakorporalen Blutkreislauf 1000 sowie, andeutungsweise, einen Dialysatkreislauf 2000 einer Behandlungsvorrichtung 3000, beispielsweise einer Hämodiafiltrationsmaschine.

Der extrakorporale Blutkreislauf 1000 weist eine medizinische Funktionseinrichtung 100, z. B. eine (Disposable-)Blutkassette, auf, ist hiermit verbunden oder zumindest abschnittsweise hierin integriert.

Die Funktionseinrichtung 100 ist an die Behandlungsvorrichtung 3000 funktional angekoppelt und wirkt mit den Pumpenantrieben, Aktoren und Sensoren der in Fig. 1 nur angedeuteten Behandlungsvorrichtung 3000 funktional zusammen. Die Pumpenantriebe, Aktoren und Sensoren der Behandlungsvorrichtung 3000 wirken mit einer Steuer- und/oder Regelungseinrichtung 27 der Behandlungsvorrichtung 3000 funktional zusammen. Sie können mit der Steuer- und/oder Regelungseinrichtung in Signalverbindung stehen.

Verbunden ist der extrakorporale Blutkreislauf 1000 mit einer Behandlungseinrichtung 200, beispielsweise einem Dialysator oder einem Blutfilter.

Im oder am extrakorporalen Blutkreislauf 1000 sind eine Blutpumpe 11 sowie eine Substituatpumpe 17 angeordnet. Die Blutpumpe 11 und die Substituatpumpe 17 können in einem später ablaufenden Behandlungsverfahren Blut bzw. Substituat fördern.

Die Blutpumpe 11 und/oder die Substituatpumpe 17 können als Fördereinrichtung(en) zum Fördern des Fluids zum Zwecke des Befüllens des extrakorporalen Blutkreislaufs 1000 eingesetzt werden. Dasselbe gilt für eine nicht in Fig. 17 dargestellte Pumpe des Dialysatkreislaufs.

Der extrakorporale Blutkreislauf 1000 weist einen arteriellen Luftblasendetektor 15 (auch als "art. ABD" bezeichnet für "arterial air bubble detector") auf. Er weist ferner einen venösen Luftblasendetektor 25 (auch als "ven. ABD" bezeichnet für "venous air bubble detector") auf. Beispielsweise kann der venöse Luftblasendetektor 25 als Erkennungseinrichtung zum Erkennen eines Füllzustandes in den extrakorporalen Blutkreislauf 1000 eingesetzt werden.

Die Erfindung dient in einigen Ausführungsformen dazu, das Fluidvolumen, welches zum Füllen des extrakorporalen Blutkreislaufs 1000, d. h. der Blutseite des Leitungssystems, benötigt wird, durch die Behandlungsvorrichtung 3000 zu ermitteln.

In bestimmten Ausführungsformen des offenbarten, nicht-erfindungsgemäßen Verfahrens wird hierzu das Volumen des zum Befüllen des extrakorporalen Blutkreislaufs 1000 eingebrachten Fluids erfasst, das erforderlich ist, um eine Pegeldetektion (z. B. an einer venösen Kammer 26) oder eine Meldung durch den venösen Luftblasendetektor 25 auszulösen. Das erfasste Volumen kann z. B. durch Ermitteln der benötigten Rotordrehungen der Blutpumpe 11 und/oder der Substituatpumpe 17 oder durch Wägung eines Flüssigkeitsbeutels mit frischer Spül-/Substituatlösung bestimmt werden. Das erfasste Volumen kann mit einem Faktor multipliziert werden, um das benötigte Spülvolumen angeben oder einstellen zu können.

Fällt der Flüssigkeitspegel an der Detektionsstelle, d. h. am Erkennungsort oder an der Erkennungseinrichtung, während des Befüllens wieder ab, z. B. weil sich Restluft aus der Behandlungseinrichtung 200 löst, kann das zum erneuten Anheben des Pegels benötigte Volumen zum bereits ermittelten Volumen hinzuaddiert werden. Das weitere Verwenden des derart erfassten kumulierten Volumens kann dabei vorteilhaft die Genauigkeit der Bestimmung des erfassten Volumens erhöhen. Das ermittelte Volumen wird in bestimmten Ausführungsformen verwendet zur Identifikation eines verwendeten Blutschlauchsatzes. Die Identifikation des Blutschlauchsatzes kann z. B. anhand einer Vergleichstabelle erfolgen, in der typische Füllvolumina für verschiedene Disposablekonfigurationen oder Blutschlauch/Blutfilter-Kombinationen hinterlegt sind.

Die Vergleichstabelle ist in einigen erfindungsgemäßen Ausführungsformen innerhalb der Steuer- und/oder Regelungseinrichtung 27 in einem Datenspeicher gespeichert oder kann erfindungsgemäß in diesem gespeichert werden.

Die Steuer- und/oder Regelungseinrichtung 27 weist in manchen erfindungsgemäßen Ausführungsformen eine Einrichtung zum Auswerten von Messdaten und Berechnen eines Fördervolumens, insbesondere des kumulierten Fördervolumens aus den Messdaten, auf. Sie kann zusätzlich eine Einrichtung zum Vergleichen des berechneten Fördervolumens mit den Daten aus der Vergleichstabelle aufweisen, zudem eine Einrichtung zum Zuordnen des berechneten Fördervolumens zu bestimmten vorgegebenen Blutschlauchsätzen aus der Vergleichstabelle. Die vorgenannten Einrichtungen können in einer einzigen Einrichtung vereint vorliegen.

So können z. B. vorteilhaft einfach unterschiedliche Blutschlauchsysteme für z. B. pädiatrische und Erwachsenen-Dialyse oder unterschiedliche Behandlungsverfahren (Single-Needle/Double-Needle) unterschieden werden. In Abhängigkeit des identifizierten Disposabletyps können an der Behandlungsvorrichtung, insbesondere automatisch und ohne Zutun der Aufsichtsperson, Behandlungsmodalitäten gesperrt und/oder Behandlungsparameter eingeschränkt werden.

In weiteren Ausführungsformen wird das ermittelte Volumen verwendet zur Identifikation der eingesetzten Behandlungseinrichtung 200, z. B. eines eingesetzten Dialysators.

Zur Verfeinerung der Identifikation können weitere (ergänzende) und der Behandlungsvorrichtung zur Verfügung stehende charakteristische Merkmale hinzugezogen werden (z. B. ein dialysatseitiges Füllvolumen, blut- und/oder dialysatseitige Strömungswiderstände, der Transmembrandruck und dergleichen).

In Fig. 1 sind ferner eine arterielle Blutschlauchklemme 29 sowie eine venöse Blutschlauchklemme 31 dargestellt.

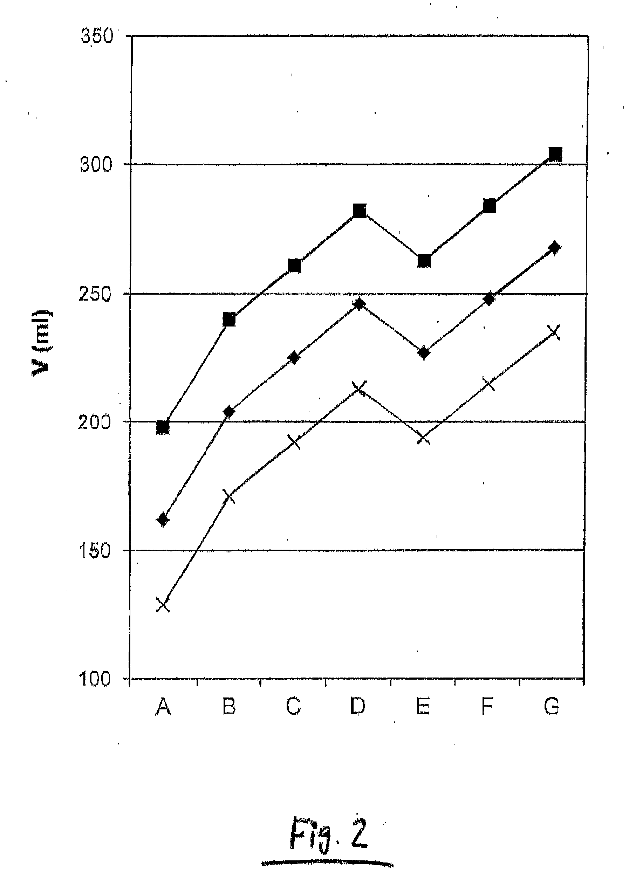

**Fig. 2** zeigt verschiedene Füllvolumina V (in ml) eines ersten extrakorporalen Blutkreislaufs für eine Single-Needle-Behandlung (ihm zugehörige Werte sind mit Quadraten markiert), eines zweiten extrakorporalen Blutkreislaufs für eine Double-Needle-Behandlung (ihm zugehörige Werte sind mit Rauten markiert), und eines dritten extrakorporalen Blutkreislaufs für eine Blutkassette (ihm zugehörige Werte sind mit Kreuzen markiert).

Das Füllvolumen des ersten Blutkreislaufs (Quadrate) beträgt 166 ml. Das Füllvolumen des zweiten Blutkreislaufs (Rauten) beträgt 130 ml. Das Füllvolumen des dritten Blutkreislaufs (Kreuze) beträgt 97 ml. Diese Angaben gelten jeweils für den Blutkreislauf, solange dieser nicht mit einem Blutfilter verbunden ist.

Fig. 2, zeigt, wie sich das Gesamtfüllvolumen der o. g. Blutkreisläufe erhöht, und unterschiedlich erhöht, sobald diese mit einem der Filter A, B, C, D, E, F oder G verbunden sind.

Aus Fig. 2 ist somit gut zu sehen, dass die Kenntnis des Füllvolumens eines konkreten Blutkreislaufs bereits erkennen lässt, mit welchem Filter dieser im Moment seines Befüllens im Sinne der Erfindung verbunden ist.

In bestimmten Ausführungsformen des offenbarten, nicht-erfindungsgemäßen Verfahrens umfasst das Verfahren das Festlegen von Parametern oder Parameterwerten zur Verfahrenssteuerung in der nachfolgenden Behandlung, z. B. Festlegung maximal/minimal zulässiger Pumpenraten (kleine Dialysatoren haben kleinere empfohlene Blutflüsse als größere), automatische Festlegung von Reinfusionsvolumina, o. ä.

Tabelle 1, welche dies widerspiegelt, ist im Folgenden für den minimalen Fluss min_Flow und den maximalen Fluss max_Flow für die o. g. Blutfilter A bis G, welche unterschiedliche Fassungs- oder Blutvolumen haben, gezeigt.

Tabelle 1

| Blutfilter | Blutvolumen | min_Flow | max_Flow |
|---|---|---|---|
| A | 32 | 50 | 200 |
| B | 74 | 150 | 400 |
| C | 95 | 200 | 500 |
| D | 116 | 250 | 600 |
| E | 97 | 150 | 400 |
| F | 118 | 200 | 500 |
| G | 138 | 300 | 600 |

Für den Fachmann ist somit erkennbar, dass die Kenntnis des verwendeten Filtertyps, welcher mittels des offenbarten, nicht-erfindungsgemäßen Verfahrens ermittelt werden kann, auch für die Steuerung der Behandlungsvorrichtung während einer sich anschließenden Behandlung genutzt werden kann. So kann ein minimaler oder ein maximaler Fluss (Rate) min Flow oder max_Flow, welcher vom Typen des Blutfilters abhängt, automatisch eingestellt werden.

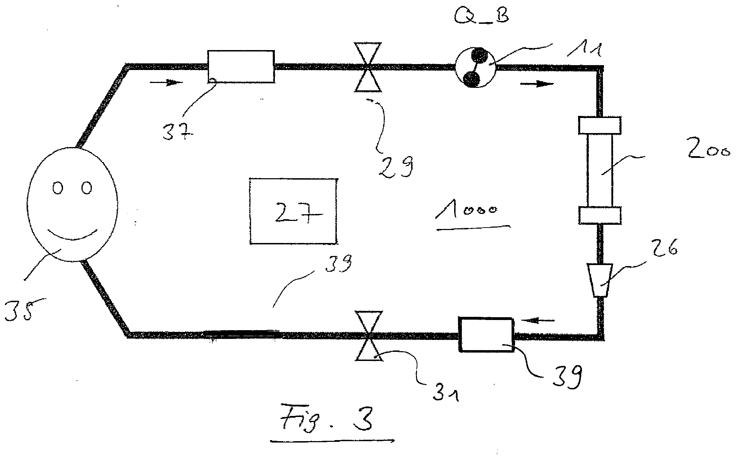

**Fig. 3** zeigt eine schematische Abbildung eines weiteren extrakorporalen Blutkreislaufs 1000. Das offenbarte, nicht-erfindungsgemäße Verfahren kann am extrakorporalen Blutkreislaufs 1000 beispielsweise wie folgt ausgeführt werden. Dabei wird angenommen, dass die Blutreinigungseinrichtung 200 ein Dialysator ist:

Nachdem ein sehr schematisch dargestellter und mit dem Bezugzeichen 35 bezeichneter Patient mit dem extrakorporalen Blutkreislauf 1000 konnektiert ist, läuft die Blutpumpe 11 mit einem konstanten Blutfluss Q_B (ml/min) an. Das Blut strömt an einem ersten Sensor 37 vorbei über die arterielle Blutschlauchklemme 29 des arteriellen Schenkels in den extrakorporalen Blutkreislauf 1000 ein.

Das einströmende Blut wird dabei vom ersten Sensor 37 (beispielsweise einem optischer Sensor, beispielsweise einem optischen Sensor zur Messung wenigstens eines Blutparameters) zu einem ersten Zeitpunkt t1 (sec) erkannt.

Zu einem zweiten Zeitpunkt t2 (sec) wird das Blut im venösen Schenkel mittels eines zweiten Sensors 39, beispielsweise einem optischen Sensor, erkannt, sofern es denn dort (bereits) vorliegt. Der Nachweis von Blut mittels des zweiten Sensors 39 kann rein exemplarisch mittels Infrarot-Transmissionsmessung erfolgen.

Der zweite Sensor 39 kann in bestimmten erfindungsgemäßen Ausführungsformen mit einer Einrichtung zum Erkennen von Luftblasen verbunden sein oder in einer gemeinsamen Baugruppe vorliegen. Er ist in manchen erfindungsgemäßen Ausführungsformen stromaufwärts der venösen Blutschlauchklemme 31 angeordnet. In gewissen erfindungsgemäßen Ausführungsformen ist er stromabwärts der venösen Kammer 26 bzw. der venösen Blasenkammer angeordnet.

Aus dem konstanten Blutfluss Q_B (ml/min) und der Zeitdifferenz zwischen dem ersten Zeitpunkt t1 (sec) und dem zweiten Zeitpunkt t2 (sec) kann das Volumen *V_Set* des gesamten extrakorporalen Blutkreislaufs 1000 wie folgt ermittelt werden:

$$(1) \quad V\_Set = (t2 - t1) \ x \ Q\_B \ / \ 60 \ (in \ ml)$$

Aufgrund der bekannten Beziehung

$$(2) \quad V\_Set = V\_Dial + V\_Schlauch$$

mit *V_Set* für das Volumen des gesamten Blutschlauchsatzes oder -sets, kann falls das Schlauchvolumen V_*Schlauch* bekannt ist, das Volumen *V_Dial* des Dialysators berechnet werden nach

$$(3) \quad V\_Dial = V\_Set - V\_Schlauch.$$

Bauteile, die im extrakorporalen Blutkreislauf der Fig. 1 dargestellt sind, kann auch der extrakorporale Blutkreislauf der Fig. 3 aufweisen, und umgekehrt.

**Bezugszeichenliste**

| Bezugszeichen | Bezeichnung |
|---|---|
| 1000 | Extrakorporaler Blutkreislauf |
| 2000 | Dialysatkreislauf |
| 3000 | Behandlungsvorrichtung |
| 100 | Medizinische Funktionseinrichtung |
| 200 | Behandlungseinrichtung |
| 11 | Blutpumpe |
| 15 | arterieller Luftblasendetektor |
| 17 | Substituatpumpe |
| 25 | venöser Luftblasendetektor |
| 26 | venöse Kammer |

(fortgesetzt)

| Bezugszeichen | Bezeichnung |
|---|---|
| 27 | Steuer- und/oder Regelungseinrichtung |
| 29 | arterielle Blutschlauchklemme |
| 31 | venöse Blutschlauchklemme |
| 35 | Patient |
| 37 | optischer Sensor |
| 39 | optischer Sensor |
| P | Druckmesseinrichtungen |

**Patentansprüche**

1. Steuer- oder Regelungseinrichtung (27), konfiguriert zur Durchführung eines Verfahrens zum Ermitteln wenigstens eines Parameters eines extrakorporalen Blutkreislaufs (1000), wobei der extrakorporale Blutkreislauf (1000) eine Behandlungseinrichtung (200) aufweist, mit den Schritten:

   - Befüllen eines extrakorporalen Blutkreislaufs (1000) durch Einbringen eines Fluids, Fördern des Fluides durch den extrakorporalen Blutkreislauf (1000) oder Abschnitte hiervon, mittels einer Blutpumpe (11) bei konstantem und/oder vorbestimmtem Fluss ($Q\_B$) der Blutpumpe (11), und Bestimmen oder Errechnen einer Laufzeit des mittels der Blutpumpe (11) in den extrakorporalen Blutkreislauf (1000), oder Abschnitte hiervon, geförderten Fluids; und
   - Erfassen, Ermitteln oder Berechnen der Größe eines Volumens des eingebrachten Fluids, welches zum Befüllen des extrakorporalen Blutkreislaufs (1000) oder eines definierten oder vorbestimmten Abschnitts hiervon erforderlich ist;
   - Weiterverarbeiten der Größe des erfassten Volumens des Fluids zum Erhalt des wenigstens einen Parameters des extrakorporalen Blutkreislaufs (1000);

   wobei der Parameter eine Angabe ist, welche den extrakorporalen Blutkreislauf unterscheidbar macht, seine Größe angibt, seine Typenzugehörigkeit, seine Klassenzugehörigkeit, seine Eignung für bestimmte Behandlungsmodalitäten oder -verfahren oder seine Eignung zur Verwendung mit bestimmten Behandlungsvorrichtungen angibt oder Aussagen über einzelne Komponenten hiervon macht,
   **dadurch gekennzeichnet, dass**
   das Verfahren den Schritt umfasst:
   Vergleichen des ermittelten Parameters mit Parametern einer Vielzahl einsetzbarer Behandlungseinrichtungen zur Identifikation der verwendeten Behandlungseinrichtung (200).

2. Steuer- oder Regelungseinrichtung (27) nach Anspruch 1, wobei das Fluid durch die Blutseite des Leitungssystems des extrakorporalen Blutkreislaufs (1000) gefördert wird.

3. Steuer- oder Regelungseinrichtung (27) nach einem der Ansprüche 1 bis 2, wobei das Befüllen nach dem Konnektieren eines Patienten (35) mit dem extrakorporalen Blutkreislauf (1000) erfolgt.

4. Steuer- oder Regelungseinrichtung (27) nach einem der vorangegangenen Ansprüche, wobei das in den extrakorporalen Blutkreislauf geförderte Fluid Blut ist.

5. Steuer- oder Regelungseinrichtung (27) nach einem der vorangegangenen Ansprüche, wobei das Volumen des Fluids ein kumuliertes Volumen ist.

6. Steuer- oder Regelungseinrichtung (27) nach einem der vorangegangenen Ansprüche, wobei das Verfahren die Schritte umfasst:

   - Fördern des Fluids durch den extrakorporalen Blutkreislauf (1000) oder Abschnitte hiervon bei konstantem und/oder vorbestimmtem Fluss ($Q\_B$) der Blutpumpe (11);

- Ermitteln des Zeitpunkts des Auftretens des Fluids an einem ersten Sensor (37) des extrakorporalen Blutkreislaufs (1000) als ersten Zeitpunkt (t1);
- Ermitteln des Zeitpunkts des Auftretens des Fluids an einem zweiten Sensor (39) des extrakorporalen Blutkreislaufs (1000) als zweiten Zeitpunkt (t2);
- Ermitteln der Zeitdifferenz zwischen dem ersten Zeitpunkt (t1) und dem zweiten Zeitpunkt (t2);

wobei das Erfassen, Ermitteln oder Berechnen der Größe des Volumens des eingebrachten Fluids eine Multiplikation der Zeitdifferenz mit dem konstanten Fluss (Q_B) der Blutpumpe (11) ist oder umfasst.

7. Steuer- oder Regelungseinrichtung (27) nach Anspruch 6, wobei der erste Sensor (37) und/oder der zweite Sensor (39) als optische Sensoren ausgestaltet sind.

8. Steuer- oder Regelungseinrichtung (27) nach Anspruch 6, wobei der erste Sensor (37) und/oder der zweite Sensor (39) als Sensoren ausgestaltet sind, welche mittels Ultraschallmessung, Akustikmessung oder Temperaturmessung die Anwesenheit von Fluid feststellen.

9. Steuer- oder Regelungseinrichtung (27) nach einem der Ansprüche 6 bis 8, wobei der erste Sensor (37) und/oder der zweite Sensor (39) optische Sensoren zur Messung wenigstens eines Blutparameters sind.

10. Steuer- oder Regelungseinrichtung (27) nach einem der Ansprüche 6 bis 9, wobei der erste Sensor (37) stromaufwärts einer arteriellen Blutschlauchklemme (29) und der zweite Sensor (39) stromaufwärts einer venösen Blutschlauchklemme (31) und/oder stromabwärts einer venösen Kammer (26) oder einer venösen Blasenkammer angeordnet ist.

11. Steuer- oder Regelungseinrichtung (27) nach einem der vorangegangenen Ansprüche, wobei die Erfassung mittels einer Erfassungseinrichtung erfolgt, welche wenigstens eine Erkennungseinrichtung zum Erkennen eines Fluids oder eines Fluidzustands aufweist oder hiermit verbunden ist, wobei die Erkennungseinrichtung ausgewählt ist aus der Gruppe, welche aus Pegeldetektoren, Luftdetektoren (15, 25), optische Detektoren, Ultraschalldetektoren, Schalldetektoren oder beliebigen Kombinationen hiervon besteht.

12. Steuer- oder Regelungseinrichtung (27) nach Anspruch 11, wobei die Erkennungseinrichtung im oder am extrakorporalen Blutkreislauf angeordnet ist und/oder mit diesem in Wirk- oder Signalverbindung steht.

13. Steuer- oder Regelungseinrichtung (27) nach einem der vorangegangenen Ansprüche, wobei das Erfassen oder Bestimmen des Volumens mittels der Steuer- oder Regelungseinrichtung (27) automatisch erfolgt.

14. Steuer- oder Regelungseinrichtung (27) nach einem der vorangegangenen Ansprüche, wobei die Größe des Volumens des Fluids über die Rotordrehungen der Blutpumpe (11) zum Fördern des Fluids erfasst wird.

15. Steuer- oder Regelungseinrichtung (27) nach einem der vorangegangenen Ansprüche, wobei das Verfahren den Schritt umfasst:

- Festlegen einer Steuerung oder von Flussraten (min_Flow, max_Flow) des Behandlungsverfahrens unter Einbezug oder in Abhängigkeit des ermittelten Parameters.

16. Steuer- oder Regelungseinrichtung (27) nach einem der vorangegangenen Ansprüche, wobei das Verfahren den Schritt umfasst:

- Sperren von Behandlungsmodalitäten und/oder Einschränken von Behandlungsparametern des Behandlungsverfahrens bei Verwendung des extrakorporalen Blutkreislaufs (1000) in Abhängigkeit des ermittelten Parameters.

17. Steuer- oder Regelungseinrichtung (27) nach einem der vorangegangenen Ansprüche, wobei das Verfahren den Schritt umfasst:

- Festlegen von Reinfusionsvolumina unter Einbezug oder in Abhängigkeit des ermittelten Parameters.

18. Behandlungsvorrichtung, welche eine Steuer- oder Regelungseinrichtung (27) gemäß einem der Ansprüche 1 bis 17 aufweist.

**19.** Behandlungsvorrichtung nach Anspruch 18, welche als Dialysevorrichtung, Hämodialysevorrichtung, Hämofiltrationsvorrichtung, oder Hämodiafiltrationsvorrichtung ausgestaltet ist.

**Claims**

**1.** A control or regulating device (27) configured to carry out a method for determining at least one parameter of an extracorporeal blood circuit (1000), wherein the extracorporeal blood circuit (1000) comprises a treatment apparatus (200), including the following steps:

- filling an extracorporeal blood circuit (1000) by introducing a fluid, conveying the fluid through the extracorporeal blood circuit (1000) or portions thereof by means of a blood pump (11) at a constant and/or predetermined flow (Q_B) of the blood pump (11),
and determining or calculating a running time of the fluid conveyed into the extracorporeal blood circuit (1000), or portions thereof, by means of the blood pump (11); and
- recording, determining or calculating the size of a volume of the introduced fluid required for filling the extracorporeal blood circuit (1000) or a defined or predetermined portion thereof;
- further processing of the size of the recorded volume of the fluid to obtain the at least one parameter of the extracorporeal blood circuit (1000);

wherein the parameter is an indication, which distinguishes the extracorporeal blood circuit, indicates its size, its belonging to a type, its belonging to a class, its suitability for specific treatment modalities or methods or its suitability for use with specific treatment apparatuses or makes statements about individual components thereof,
**characterized in that**
the method comprises the step of:
comparing the determined parameter with parameters of a plurality of usable treatment apparatuses to identify the treatment apparatus (200) in use.

**2.** The control or regulating device (27) according to claim 1, wherein the fluid is conveyed through the line system's blood side of the extracorporeal blood circuit (1000).

**3.** The control or regulating device (27) according to anyone of claims 1 to 2, wherein the filling takes place after connecting a patient (35) to the extracorporeal blood circuit (1000).

**4.** The control or regulating device (27) according to anyone of the preceding claims, wherein the fluid conveyed through the extracorporeal blood circuit is blood.

**5.** The control or regulating device (27) according to anyone of the preceding claims, wherein the volume of the fluid is a cumulative volume.

**6.** The control or regulating device (27) according to anyone of the preceding claims, wherein the method comprises the steps of:

- conveying the fluid through the extracorporeal blood circuit (1000) or portions thereof at a constant and/or predetermined flow (Q_B) of the blood pump (11);
- determining the point in time of occurrence of the fluid at a first sensor (37) of the extracorporeal blood circuit (1000) as first point in time (t1);
- determining the point in time of occurrence of the fluid at a second sensor (39) of the extracorporeal blood circuit (1000) as second point in time (t2);
- determining the time difference between the first point in time (t1) and the second point in time (t2);

wherein the detection, determination or calculation of the size of the volume of the introduced fluid is or comprises a multiplication of the time difference with the constant flow (Q_B) of the blood pump (11).

**7.** The control or regulating device (27) according to claim 6, wherein the first sensor (37) and/or the second sensor (39) are designed as optical sensors.

8. The control or regulating device (27) according to claim 6, wherein the first sensor (37) and/or the second sensor (39) are designed as sensors which determine the presence of fluid by means of ultrasonic measurement, acoustic measurement or temperature measurement.

9. The control or regulating device (27) according to anyone of claims 6 to 8, wherein the first sensor (37) and/or the second sensor (39) are optical sensors for measuring at least one blood parameter.

10. The control or regulating device (27) according to anyone of claims 6 to 9, wherein the first sensor (37) is arranged upstream of an arterial blood tube clamp (29) and the second sensor (39) is arranged upstream of a venous blood tube clamp (31) and/or downstream of a venous chamber (26) or a venous bubble chamber.

11. The control or regulating device (27) according to anyone of the preceding claims, wherein the recording is carried out by means of a recording device which has or is connected to at least one detection device for detecting a fluid or a fluid state, the detection device being selected from the group consisting of level detectors, air detectors (15, 25), optical detectors, ultrasonic detectors, sound detectors or any combinations thereof.

12. The control or regulating device (27) according to claim 11, wherein the detection device is arranged in or on the extracorporeal blood circuit and/or is in operative or signal connection with it.

13. The control or regulating device (27) according to anyone of the preceding claims, wherein the recording or determination of the volume by means of the control or regulating device (27) is carried out automatically.

14. The control or regulating device (27) according to anyone of the preceding claims, wherein the size of the volume of the fluid is recorded via the rotor rotations of the blood pump (11) for conveying the fluid.

15. The control or regulating device (27) according to anyone of the preceding claims, wherein the method comprises the step of:

   - determining a control or flow rates (min_Flow, max_Flow) of the treatment method taking into account or depending on the determined parameter.

16. The control or regulating device (27) according to anyone of the preceding claims, wherein the method comprises the step of:

   - blocking treatment modalities and/or restricting treatment parameters of the treatment method by using the extracorporeal blood circuit (1000) depending on the determined parameter.

17. The control or regulating device (27) according to anyone of the preceding claims, wherein the method comprises the step of:

   - determining reinfusion volumes taking into account or depending on the determined parameter.

18. A treatment apparatus, which comprises a control or regulating device (27) according to anyone of claims 1 to 17.

19. The treatment apparatus according to claim 18, which is designed as a dialysis apparatus, hemodialysis apparatus, hemofiltration apparatus, or hemodiafiltration apparatus.

**Revendications**

1. Un dispositif de commande ou de régulation (27) configuré pour exécuter un procédé de détermination d'au moins un paramètre d'un circuit sanguin extracorporel (1000),
où le circuit sanguin extracorporel (1000) comprend un appareil de traitement (200) comportant les étapes suivantes:

   - le remplissage d'un circuit sanguin extracorporel (1000) par l'introduction d'un fluide, l'acheminement du fluide au travers du circuit sanguin extracorporel (1000) ou de sections de celui-ci au moyen d'une pompe à sang (11) à un débit constant et/ou prédéterminé (Q_B) de la pompe à sang (11), ainsi que la détermination ou le calcul d'un temps de circulation du fluide acheminé au moyen de la pompe à sang (11) dans le circuit sanguin extracorporel (1000), ou dans des sections de celle-ci; et

- l'enregistrement, la détermination ou le calcul de la taille d'un volume du fluide introduit nécessaire au remplissage du circuit sanguin extracorporel (1000) ou d'une section définie ou prédéterminée de celui-ci;
- la poursuite du traitement de la taille du volume du fluide enregistrée pour obtenir au moins un paramètre du circuit sanguin extracorporel (1000);

où le paramètre est une indication, qui permet de distinguer le circuit sanguin extracorporel, qui indique sa taille, son appartenance à un type, son appartenance à une classe, son aptitude à des modalités ou des procédés de traitement spécifiques ou son aptitude à être utilisé avec des appareils de traitement spécifiques ou qui fournit des informations sur ses composants individuels,

**caractérisé en ce que**

le procédé comprend l'étape consistant à:

comparer le paramètre déterminé avec des paramètres d'une pluralité d'appareils de traitements utilisables afin d'identifier l'appareil de traitement (200) utilisé.

2. Le dispositif de commande ou de régulation (27) selon la première revendication, où le fluide est acheminé au travers du côté sanguin du système de conduits du circuit sanguin extracorporel (1000).

3. Le dispositif de commande ou de régulation (27) selon l'une quelconque des revendications 1 à 2, où le remplissage a lieu après le raccordement d'un patient (35) au circuit sanguin extracorporel (1000).

4. Le dispositif de commande ou de régulation (27) selon l'une quelconque des revendications précédentes, où le fluide acheminé dans le circuit sanguin extracorporel est du sang.

5. Le dispositif de commande ou de régulation (27) selon l'une quelconque des revendications précédentes, où le volume du fluide est un volume cumulé.

6. Le dispositif de commande ou de régulation (27) selon l'une quelconque des revendications précédentes, où le procédé comprend les étapes consistant à:

- acheminer le fluide au travers du circuit sanguin extracorporel (1000) ou de sections de celui-ci à un débit constant et/ou prédéterminé (Q_B) de la pompe à sang (11) ;
- déterminer l'instant de l'apparition du fluide au niveau d'un premier capteur (37) du circuit sanguin extracorporel (1000) en tant que premier instant (t1);
- déterminer l'instant de l'apparition du fluide au niveau d'un second capteur (39) du circuit sanguin extracorporel (1000) en tant que second instant (t2);
- déterminer la différence de temps entre le premier instant (t1) et le second instant (t2);

où la détection, la détermination ou le calcul de la taille du volume du fluide introduit est ou inclut une multiplication de la différence de temps par le débit constant (Q_B) de la pompe à sang (11).

7. Le dispositif de commande ou de régulation (27) selon la revendication 6, où le premier capteur (37) et/ou le second capteur (39) sont conçus comme des capteurs optiques.

8. Le dispositif de commande ou de régulation (27) selon la revendication 6, où le premier capteur (37) et/ou le second capteur (39) sont conçus comme des capteurs qui déterminent la présence de fluide au moyen d'une mesure par ultrasons, d'une mesure acoustique ou d'une mesure de température.

9. Le dispositif de commande ou de régulation (27) selon l'une quelconque des revendications 6 à 8, où le premier capteur (37) et/ou le second capteur (39) sont des capteurs optiques pour la mesure d'au moins un paramètre sanguin.

10. Le dispositif de commande ou de régulation (27) selon l'une quelconque des revendications 6 à 9, où le premier capteur (37) est agencé en amont d'une pince de serrage de tuyau sanguin artériel (29) et où le second capteur (39) est agencé en amont d'une pince de serrage de tuyau sanguin veineux (31) et/ou en aval d'une chambre veineuse (26) ou d'une chambre à bulles veineuse.

11. Le dispositif de commande ou de régulation (27) selon l'une quelconque des revendications précédentes, où la détection s'effectue au moyen d'un dispositif de détection comportant ou étant relié à au moins un dispositif de détection permettant de détecter un fluide ou un état de fluide, le dispositif de détection étant choisi depuis le groupe constitué de détecteurs de niveau, de détecteurs d'air (15, 25), de détecteurs optiques, de détecteurs à ultrasons, de détecteurs sonores ou de quelconques combinaisons de ceux-ci.

**12.** Le dispositif de commande ou de régulation (27) selon la revendication 11, où le dispositif de détection est agencé dans ou sur le circuit sanguin extracorporel et/ou est relié à celui-ci par liaison active ou par liaison de signal.

**13.** Le dispositif de commande ou de régulation (27) selon l'une quelconque des revendications précédentes, où la saisie ou la détermination du volume s'effectue automatiquement au moyen du dispositif de commande ou de régulation (27).

**14.** Le dispositif de commande ou de régulation (27) selon l'une quelconque des revendications précédentes, où la taille du volume du fluide est saisie via les rotations du rotor de la pompe à sang (11) pour l'acheminement du fluide.

**15.** Le dispositif de commande ou de régulation (27) selon l'une quelconque des revendications précédentes, le procédé comprenant l'étape consistant à:

- déterminer une commande ou des débits (min_Flow, max_Flow) du procédé de traitement en tenant compte ou en fonction du paramètre déterminé.

**16.** Le dispositif de commande ou de régulation (27) selon l'une quelconque des revendications précédentes, où le procédé comprend l'étape consistant à:

- bloquer des modalités de traitement et/ou restreindre des paramètres de traitement du procédé de traitement utilisant le circuit sanguin extracorporel (1000) en fonction du paramètre déterminé.

**17.** Le dispositif de commande ou de régulation (27) selon l'une quelconque des revendications précédentes, où le procédé comprend l'étape consistant à:

- déterminer des volumes de ré-infusion en tenant compte ou en fonction du paramètre déterminé.

**18.** Un appareil de traitement qui comprend un dispositif de commande ou de régulation (27) selon l'une quelconque des revendications 1 à 17.

**19.** L'appareil de traitement selon la revendication 18 conçu comme un appareil de dialyse, un appareil d'hémodialyse, un appareil d'hémofiltration ou un appareil d'hémodiafiltration.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0416808 A1
- WO 2010133319 A
- EP 1748292 A1
- WO 2004057313 A1

**In der Beschreibung aufgeführte Nicht-Patentliteratur**

- **KRIVITSKI et al.** In vivo measurement of hemodialyzer fiber bundle volume: theory and validation. *Kidney International,* 1998, vol. 54 (5), 1751-1758